# EUROPÄISCHE PATENTSCHRIFT

(11) **EP 0 537 452 B1**
(45) Veröffentlichungstag und Bekanntmachung des Hinweises auf die Patenterteilung: **13.09.1995**
(21) Anmeldenummer: 92114596.7
(22) Anmeldetag: 27.08.1992
(51) Int. Cl.: A61B 17/28

(54) **Medizinische Zange**
Surgical forceps
Pince chirurgicale

(30) Priorität: 19.09.1991 DE 4131176
(43) Veröffentlichungstag der Anmeldung: 21.04.1993
(73) Patentinhaber: Richard Wolf GmbH, 75438 Knittlingen (DE)
(72) Erfinder: Boedel, Manfred, W-7136 Oetisheim (DE); Metsch, Dieter, W-7527 Kraichtal/Bahnbrücken (DE)
(74) Vertreter: Wilcken, Thomas, Dipl.-Ing.

(56) Entgegenhaltungen:
- DE-A- 3 601 166
- DE-A- 3 709 067
- DE-A- 3 741 879
- DE-A- 3 819 123
- DE-A- 4 115 937

## Beschreibung

Die Erfindung geht aus von einer medizinischen Zange mit distalen Maulteilen, von denen mindestens ein Maulteil gegen das andere mittels einer proximalen Handhabe beweglich betätigbar ist, wobei die bei Betätigung der Handhabe ausgeübte Kraft über eine axial verschiebbare Stange auf das bewegliche Maulteil übertragbar ist und wobei ein Überlastungsschutz vorgesehen ist, der im Falle einer Überlastung mit Bruchgefahr wirksam wird und die Schließkraft der Maulteile auf einen vorgegebenen Wert begrenzt.

Medizinische Zangen der vorgenannten Art mit schneidenden, stanzenden oder klemmenden und haltenden distalen Maulteilen müssen gegen Überlastung und Bruchgefahr geschützt sein, zumal die durch die Handhabe auf die distalen Maulteile ausgeübte Kraft infolge der vorhandenen Hebelübersetzungen verhältnismäßig groß sein kann.

Bei einer bekannten Ausführung solcher Zangen erreicht man den Überlastungsschutz durch Sicherungen, die in der Handhabe angebracht sind, wie z. B. gemäß der DE 36 01 166 C2 durch eine Feder, die nach einem ersten Ausführungsbeispiel den schwenkbeweglich angeordneten zweiteiligen Zangengriff und nach einem zweiten Ausführungsbeispiel die mit dem verschwenkbaren Zangenmaulteil verbundene Zug- und Druckstange federvorgespannt überbrückt und bei Überschreiten der Bruchkraft die weitere auf die beiden Zangengriffe ausgeübte Druckraft aufnimmt.

Bei beiden Ausführungen wird bei Erreichen einer bestimmten Kraft zwar nur das bewegliche Griffteilstück der Handhabe weiter ausgelenkt und das Maulteil nicht mehr bewegt, jedoch nimmt die auf die Maulteile ausgeübte Kraft entsprechend der Federkennlinie weiter zu. Darüberhinaus ist für den Operateur nicht deutlich erfühlbar, in welcher Betätigungsstellung die Betätigungskraft die Federvorspannung übersteigt, da dieser Übergang fließend ist. Hieraus können Unsicherheiten bei der Handhabung entstehen.

Bei der DE 37 09 067 A1 ist zwischen dem beweglichen Griffteil und der Zug- und Druckstange eine Ausrückkupplung vorgesehen, wobei ein in einem Griffteil federnd gelagertes Kupplungsteil in eine Ausnehmung der Stange eingreift und ausrastet, wenn eine bestimmte Kraft überschritten wird. Dieser Übergang ist nicht fließend, sondern eher abrupt, wobei der bewegliche Griffteil von der Stange total abgekuppelt wird. Auch diese Ausführung birgt Unsicherheiten in der Handhabung.

Es ist Aufgabe der Erfindung, einen Überlastungsschutz für medizinische Zangen der genannten Art zu finden, der sicher wirkt und dem Operateur ein gefühlvolles Arbeiten ermöglicht, wobei vom Operateur der Zeitpunkt des Eintritts der Überlastung fühlbar sein soll.

Entsprechend dieser Aufgabe besteht die Erfindung darin, daß bei Erreichen des Überlastzustandes die Stange oder zumindest ein Teil der Stange seitlich auslenkbar und mit einem ortsfesten Teil der Zange in formschlüssigen, eine weitere Betätigung der Zangenhandhabe sperrenden Eingriff bringbar ist.

Dadurch wird erreicht, daß bei Überlastung sowohl die Stange und das bewegliche Maulteil als auch die Handhabe sofort gegen weitere Bewegung arretiert wird. Dieser Zustand ist vom Operateur unmittelbar und direkt fühlbar und ermöglicht ihm eine weitaus sicherere Handhabung als bisher.

Gemäß einer weiteren Ausführungsform der Erfindung wird so vorgegangen, daß die Stange aus unter federnder Wirkung formschlüssig verbundenen Teilen besteht, von denen einer bei Erreichen des Überlastzustandes entgegen der Federwirkung radial auslenkbar ist und dabei mit dem ortsfesten Teil der Zange in Eingriff kommt. Die Federkraft ist auf die Haltbarkeit der Maulteile abgestimmt und gewährleistet somit optimale Bruchsicherung. Weitere vorteilhafte Merkmale sind in den Unteransprüchen angegeben.

Die Erfindung und ihre weitergehenden Ausführungsformen werden nachstehend anhand der Zeichnungen erläutert. Es zeigt:
- Figur 1: eine Seitenansicht der Zange im verkleinerten Maßstab,
- Figur 2: eine Schnittdarstellung entlang der Linie II-II im vergrößerten Maßstab,
- Figur 3 und 4: Schnittdarstellungen wie in Figur 2, jedoch mit anderen Ausführungsformen.

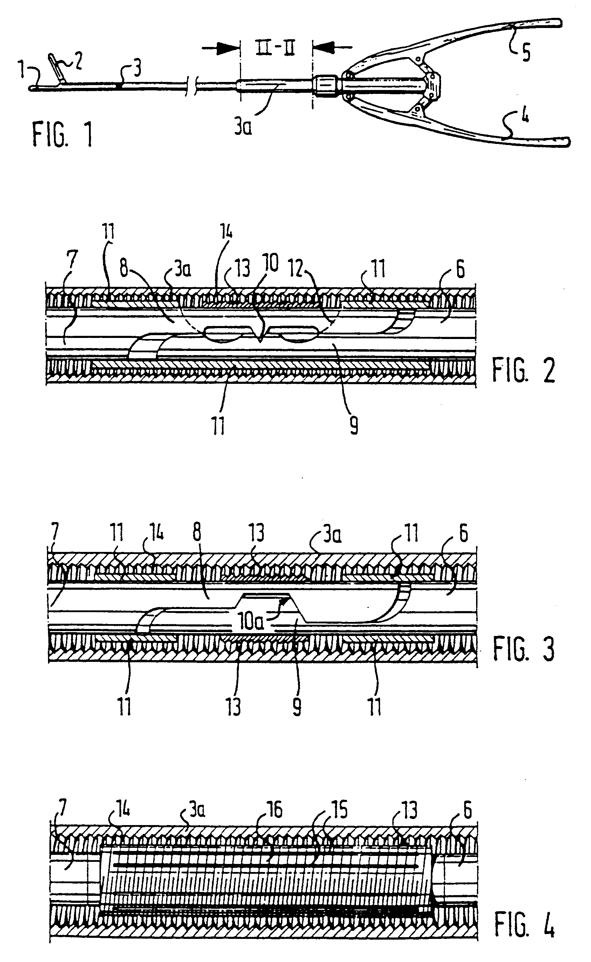

Die Zange nach Figur 1 kann als Schneid-, Halte- oder Klemmzange ausgebildet sein. Sie besteht im wesentlichen aus den Maulteilen 1 und 2, wovon im Ausführungsbeispiel das Maulteil 2 beweglich ist, und weiter aus dem Schaft 3 mit dem erweiterten Teil 3a als Zangengehäuse und der Handhabe aus scherenartig zu betätigenden Griffen 4 und 5. Von denen ist der Griff 5 mit dem Schaft 3 verbunden und der Griff 4 darin als Schwenkhebel gelagert. Durch diesen Griff 4 wird die zweiteilige Stange 6, 7 betätigt, die mit ihrem Teil 7 am beweglichen Maulteil 2 angelenkt ist und dieses bewegt.

Das Stangenteil 7 ist mit einem Formteil 8 versehen, welches formschlüssig in ein Formteil 9 eingreift, welches seinerseits mit dem Stangenteil 6 verbunden ist. Der formschlüssige Eingriff der beiden Formteile 8 und 9 ineinander erfolgt durch eine Verzahnung 10. Um die beiden Formteile 8 und 9 zusammenzuhalten bzw. zu führen, ist eine Rohrhülse 11 vorgesehen. Diese Rohrhülse 11 weist eine längliche Umfangsausnehmung 12 auf.

Die beiden der Darstellung entsprechend durch die Rohrhülse 11 zusammengehaltenen Formteile 8 und 9 werden bei normaler Betätigung des Griffes 4 gemeinsam mit den Stangenteilen 6 und 7 in gleicher Richtung und mit gleichem Weg axial verschoben, so daß das Maulteil 2 bewegt wird. Wenn das bewegliche Maulteil 2 gegen das feste Maulteil 1 zur Anlage kommt oder sonstwie an einer weiteren Bewegung gehindert wird, so daß durch die an den Griffen 4 und 5 wirkende Kraft eine Zerstörung der Maulteile oder ihrer Lagerung droht, gleiten die Flanken der Verzahnung 10 der beiden Formteile 8 und 9 aufeinander, wodurch das Formteil 8 federelastisch radial nach außen gegen den Schaftteil 3a ausgelenkt wird.

Das Formteil 8 ist auf seiner Außenseite mit einer Struktur 13, beispielsweise in Form von Gewinde- oder Zahnflanken, versehen. Diese können in eine entsprechend geformte Gegenfläche 14 an der Innenseite des Schaftteiles 3a eingreifen, wenn die Auslenkung infolge von Überlastung groß genug ist. Dadurch erfolgt ein formschlüssiger Eingriff zwischen Stangenteil 7 mit Formteil 8 und dem ortsfesten Schaftteil 3a. Dieser Eingriff sperrt die weitere Axialbewegung des Stangenteils 7 und somit auch des Stangenteils 6 in Betätigungsrichtung. Eine weitere Betätigung ist gesperrt, und somit wird eine Beschädigung der empfindlichen Maulteile 1 und 2 verhindert. Bei nachlassender Betätigungskraft nehmen die Teile selbsttätig ihre ursprüngliche Lage wieder ein.

Im Gegensatz zum Ausführungsbeispiel nach Figur 2, bei dem nur das Formteil 8 elastisch auslenkbar ist, sind bei dem durch Figur 3 dargestellten Beispiel die Formteile 8 und 9 als Bestandteile der Stangenteile 6 und 7 und durch ihre Formgebung bzw. Querschnittsverringerung federnd elastisch auslenkbar. Der formschblüssige Eingriff dieser beiden Teile miteinander muß nicht unbedingt durch mehrere Zahnflanken 10 erfolgen (wie in Figur 2), sondern kann durch zwei zusammenwirkende Flanken 10a gebildet werden. Beide Formteile 8, 9 weisen auf den Außenseiten Zahnstrukturen 13 auf, wahrend die Innenseite des Schaftteiles 3a auf einer größeren Länge ebenfalls mit einer Struktur 14 versehen ist, also angeraut, gezahnt oder mit Gewinde versehen ist. Bei einer Überlastung gleiten beide Formteile 8 und 9 als Bestandteile der Stangen 6 und 7 an den Flanken 10a radial auseinander und greifen mit der Verzahnung 13 formschlüssig in die innere Struktur 14 des Schaftteiles 3a ein, so daß also ein weiteres Verschieben der Stange und eine Überlastung der Maulteile verhindert wird. Dieser primäre Eingriff bietet noch mehr Sicherheit gegen weitere schädigende Betätigung im Überlastbereich. Um die beiden Stangenteile 6 und 7 gegeneinander zu fixieren und axial zu führen, sind zwei Rohrhülsen 11 vorgesehen.

In Figur 4 ist eine weitere mögliche Ausführungsform der Erfindung dargestellt. Der Schaftteil 3a ist wiederum mit einer inneren Verzahnung 14, Gewinde- oder Riefenstruktur versehen. Die beiden Stangenteile 6 und 7 sind durch ein mehrere, gleichmäßig auf dem Umfang verteilt angeordnete Längsschlitze 15 aufweisendes und eine äußere Verzahnung 13 oder dgl. aufweisendes Rohrelement 16 miteinander verbunden, welches eine elastische Durchmesservergrößerung erfährt, wenn die beiden Stangenteile unter zerstörerischer Krafteinwirkung gegeneinander verschoben werden.

Abweichend von den dargestellten Ausführungsbeispielen könnten mit der Zug- und Druckstange 6, 7 auch beide Maulteile 1, 2 betätigt werden. Weiterhin kann die erwähnte Stange auch einteilig ausgebildet sein, wobei im Überlastungszustand ein Bereich, z. B. der mittlere oder Endbereich, der Stange elastisch ausgelenkt ist und in Eingriff mit einem ortsfesten Zangenteil steht.

## Patentansprüche

1. Medizinische Zange mit distalen Maulteilen, von denen mindestens ein Maulteil gegen das andere mittels einer proximalen Handhabe beweglich betätigbar ist, wobei die bei Betätigung der Handhabe ausgeübte Kraft über eine axial verschiebbare Stange auf das bewegliche Maulteil übertragbar ist und wobei ein Überlastungsschutz vorgesehen ist, der im Falle einer Überlastung mit Bruchgefahr wirksam wird und die Schließkraft der Maulteile auf einen vorgegebenen Wert begrenzt, dadurch gekennzeichnet, daß bei Erreichen des Überlastzustandes die Stange oder zumindest ein Teil (8) der Stange (6, 7) seitlich auslenkbar und mit einem ortsfesten Teil (14) der Zange in formschlüssigen, eine weitere Betätigung der Zangenhandhabe sperrenden Eingriff bringbar ist.

2. Zange nach Anspruch 1, dadurch gekennzeichnet, daß die Stange (6, 7) aus formschlüssig verbundenen Teilen (8, 9) besteht, von denen zumindest einer bei Erreichen des Überlastzustandes gegen Federwirkung radial auslenkbar ist und dabei mit dem ortsfesten Teil (14) der Zange in Eingriff kommt.

3. Zange nach Anspruch 2 dadurch gekennzeichnet, daß die beiden Stangenteile (6, 7) mittels eines zylindrischen Rohrstückes (11) gegeneinander axial geführt sind und ein Teilbereich (8) einer der Stangenteile unter elastischer Verformung im Überlastzustand durch eine längliche Umfangsausnehmung (12) des zylindrischen Rohrstückes hindurchtritt und mit einer Oberfläche (13) mit einer Innenfläche (14) des Zangengehäuses (3, 3a) in formschlüssigen Eingriff gelangt.

4. Zange nach einem der vorhergehenden Ansprüche, dadurch gekennzeichnet, daß zwei einander überlappende Stangenteile (6, 7) in einem Überlappungsbereich (8, 9) mit einem verringerten Querschnitt und mehreren bei Axialverschiebung der Stangenteile gegeneinander eine radiale Auslenkung bewirkenden Zahnflanken (10a) versehen sind, wobei auf den Außenseiten angebrachte Zähne (13) bei radialer Auslenkung mit der gleichartig gezahnten Innenfläche (14) des Zangengehäuses (3, 3a) in Eingriff gelangen.

5. Zange nach Anspruch 1 dadurch gekennzeichnet, daß die Stange aus zwei Stangenteilen (6, 7), besteht, deren beide Enden in einem mehrere auf dem Umfang gleichmäßig verteilte Längsschlitze aufweisenden elastischen zylindrischen Rohrelement festgelegt sind, das bei axialer Verschiebung der beiden Stangenteile gegeneinander eine elastische Durchmesservergrößerung erfährt, wodurch die Oberfläche (13) mit einer die Innenfläche (14) des Zangengehäuses (3, 3a) zur Anlage kommt.

## Claims

1. Medical forceps with distal jaw pieces, of which at least one jaw piece is able to be actuated movably towards the other by means of a proximal handle, in which the force exerted on actuation of the handle is able to be transferred to the movable jaw piece via an axially displaceable rod and in which an overload protection is provided which becomes effective in the case of an overload with the danger of fracture and which limits the closing force of the jaw pieces to a predetermined value, characterised in that on reaching the overload state the rod or at least a part (8) of the rod (6,7) is able to be deflected laterally and is able to be brought into form fitting engagement with a fixed part (14) of the forceps, blocking a further actuation of the handle of the forceps.

2. Forceps according to Claim 1, characterised in that the rod (6,7) consists of parts (8,9) which are connected with a form fit, at least one of which, on reaching the overload state, is able to be deflected radially against the action of a spring and in so doing comes into engagement with the fixed part (14) of the forceps.

3. Forceps according to Claim 2, characterised in that the two rod parts (6,7) are guided axially towards each other by means of a cylindrical tube piece (11) and a partial region (8) of one of the rod parts passes under elastic deformation in the overload state through an elongated circumferential recess (12) of the cylindrical tube piece and comes into form fitting engagement with one surface (13) with an inner surface (14) of the forceps housing (3, 3a).

4. Forceps according to one of the preceding claims, characterised in that two rod parts (6,7) overlapping each other are provided in an overlapping region (8, 9) with a reduced cross-section and with several tooth flanks (10a) bringing about a radial deflection on axial displacement of the rod parts towards each other, in which teeth (13) arranged on the outer sides come into engagement, on radial deflection, with the similarly toothed inner surface (14) of the forceps housing (3, 3a).

5. Forceps according to Claim 1, characterised in that the rod consists of two rod parts (6, 7), the two ends of which are secured in an elastic cylindrical tube element having several elongated slots uniformly distributed on the circumference, which tube element undergoes an elastic increase in diameter on axial displacement of the two rod parts towards each other, whereby the surface (13) comes to rest against the inner surface (14) of the forceps housing (3, 3a).

## Revendications

1. Pince médicale comportant des parties distales de mâchoire, dont l'une au moins peut être actionnée, de manière à être déplacée vers l'autre partie de mâchoire, à l'aide d'une poignée proximale, et dans laquelle la force, qui est exercée lors de l'actionnement de la poignée, peut être transmise par l'intermédiaire d'une tige déplaçable axialement, à la partie de mâchoire mobile, et dans laquelle il est prévu un dispositif de protection contre une contrainte excessive, qui devient actif dans le cas d'une contrainte excessive avec risque de rupture et limite la force de fermeture des parties de mâchoire à une valeur prédéterminée, caractérisée en ce que, lorsque l'état de contrainte excessive est atteint, la tige ou au moins une partie (8) de la tige (6,7) peut être déviée latéralement et être amenée en prise avec une partie fixe (14) de la pince, selon un engrènement par formes complémentaires, qui empêche de poursuivre l'actionnement de la poignée de la pince.

2. Pince selon la revendication 1, caractérisée en ce que la tige (6,7) est formée de parties (8,9) reliées entre elles par formes complémentaires et dont l'une au moins peut être déviée radialement à l'encontre de l'action d'un ressort, lorsque l'état de contrainte excessive est atteint, et vient alors en prise avec la partie fixe (14) de la pince.

3. Pince selon la revendication 2, caractérisée en ce que les deux parties (6,7) de la tige sont guidées axialement l'une par rapport à l'autre au moyen d'un tronçon de tube cylindrique (11), et une portion (8) de l'une des parties de la tige traverse, en se déformant élastiquement, dans l'état de contrainte excessive, un évidement circonférentiel oblong (12) du tronçon de tube cylindrique et, par une surface extérieure, parvient en prise, selon un engrènement par formes complémentaires, avec une surface intérieure (14) du corps (3, 3a) de la pince.

4. Pince selon l'une des revendications précédentes, caractérisée en ce que deux parties (6,7) de la tige, qui se chevauchent mutuellement, sont dotées dans une zone de chevauchement (8,9), d'une section transversale réduite et de plusieurs flancs de dents (10a) qui provoquent une déviation radiale dans le cas d'un déplacement axial des parties de la tige l'une par rapport à l'autre, des dents (13) situées sur les faces extérieures parvenant en prise, lors d'une déviation radiale, avec la surface intérieure (14), possédant une denture identique, du corps (3,3a) de la pince.

5. Pince selon la revendication 1, caractérisée en ce que la tige est constituée de deux parties (6,7), dont les deux extrémités sont fixées dans un élément tubulaire cylindrique élastique, qui possède plusieurs fentes longitudinales réparties uniformément sur la circonférence et qui, lors d'un déplacement axial des deux parties de la tige l'une par rapport à l'autre, subit une augmentation élastique de diamètre, ce qui a pour effet que la surface extérieure (13) vient s'appliquer contre une surface intérieure (14) du corps (3,3a) de la pince.
